# EUROPÄISCHE PATENTSCHRIFT

(11) **EP 0 301 289 B1**
(45) Veröffentlichungstag und Bekanntmachung des Hinweises auf die Patenterteilung: **07.09.1994**
(21) Anmeldenummer: 88110851.8
(22) Anmeldetag: 07.07.1988
(51) Int. Cl.: C12N 15/37, C12P 21/00, G01N 33/569, C12Q 1/68

(54) **Humaner Papillomvirus Typ 41**
Humaner Papillomvirus Typ 41
Papillomavirus humain du type 41

(30) Priorität: 11.07.1987 DE 3722968
(43) Veröffentlichungstag der Anmeldung: 01.02.1989
(73) Patentinhaber: BEHRINGWERKE Aktiengesellschaft, 35001 Marburg (DE)
(72) Erfinder: Grimmel, Margitta, Dr., D-6730 Neustadt (DE); de Villiers, Ethel-Michele, Dr., D-6945 Hirschberg (DE)

(56) Entgegenhaltungen:
- NATURE, Band 321, 15. Mai 1986, Seiten 246-249; S. BEAUDENON et al.: "A novel type of human papillomavirus associated with genital neoplasias"
- INTERNATIONAL JOURNAL OF CANCER, Band 37, 1986, Seiten 61-65, Alan R. Liss,Inc.; T. KAHN et al.: "Molecular cloning and characterization of the DNAof a new human papillomavirus (HPV 30) from a laryngeal carcinoma"
- INTERNATIONAL JOURNAL OF CANCER, Band 41, Nr. 1, Januar 1988, Seiten 5-9, AlanR. Liss Inc.; M. GRIMMEL et al.: "Characterization of a new human papillomavirus (HPV 41) from disseminated warts and detection of its DNA in some skin carcinomas"

## Beschreibung

Die humanen Papillomviren (HPV) bilden eine Gruppe von etwa 40 verschiedenen Typen (zur Hausen, H. und Schneider, A. (1987), The Papillomaviruses, Howley P.M. und Salzmann, N.P. (Herausgeber), im Druck). HPV wurde im Zusammenhang mit benignen (Warzen, Kondylome im Genitalbereich) und malignen (Karzinome der Haut und der Scheide) epithelialen Neoplasmen aufgefunden. Papillomviren lassen sich nicht in Kultur vermehren. Die Verwendung des Humanen Papillomvirus Typ 41 DNA (HPV 41 DNA) als Diagnostikum sowie die Gewinnung der Expressionsprodukte, deren Verwendung als Antigene, die Isolierung von Antikörpern und die Herstellung entsprechender Diagnostika und Therapeutika setzt somit gentechnische Verfahren voraus.

Der Erfindung liegt die erstmalige Isolierung von HPV 41, eine teilweise Charakterisierung seines Genoms sowie Klonierung in pUC 19 zugrunde. Hierdurch wird ein Zugang zur Frühdiagnose von Hauttumoren eröffnet, die mit HPV 41 assoziiert sind.

Die Erfindung ist in den Patentansprüchen definiert. Weitere Ausgestaltungen der Erfindung sind im folgenden näher beschrieben.

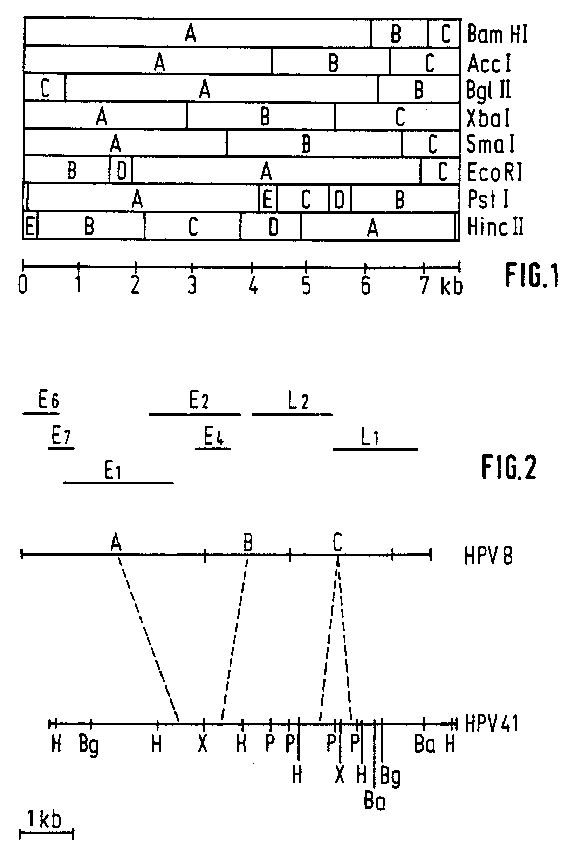

Die Klonierung von HPV 41 ermöglichte den Vergleich mit 40 anderen HPV. HPV 7, 8, 10, 17, 27, 29, 30 und 33 sind entfernt verwandt, wobei die Kolinearität von HPV 41 mit HPV 8 nachgewiesen wurde (Fig. 2) und physikalische Genomkarten für Restriktionsenzymspaltungen erstellt wurden (Fig. 1).

Damit ist ein Zugang geschaffen worden, Neoplasien, insbesondere Karzinome der Haut, auf das Vorkommen von HPV 41 zu prüfen und, über Antikörper zu HPV 41 Proteinen, gegebenenfalls therapeutisch anzugehen.

### Beispiele

1. Isolierung episomaler HPV 41 DNA
   Biopsien aus Warzengewebe von drei verschiedenen Körperarealen eines 15-jährigen Mädchens wurden unmittelbar nach Gewinnung bei -70 °C tiefgefroren und gelagert. Hochmolekulare DNA wurde daraus isoliert wie beschrieben (Gissmann et al. (1982), Int. J. Cancer 29, 143-146). Ringförmige geschlossene Doppelstrang-DNA wurde nach Radloff et al. (1967) Proc. Nat. Acad. Sci. 57, 1514-1521 aus der genannten zellulären DNA gewonnen, wobei etwa 10 µg DNA in einem 50 Ti Rotor (Beckmann Corp.) bei 45 000 Upm 48 Stunden in CsCl (Dichte 1,56 g/ml) mit Zusatz von 600 µg/ml Ethidiumbromid zentrifugiert und anschließend die Fraktionen der Dichte 1,59-1,60 gesammelt wurden. Alternativ konnte episomale HPV 41 DNA ungeschnittener DNA desselben Biopsiematerials in mit Ethidiumbromid gefärbten Agarosegelen (1 % Agarose (Seakem ME) in 40 mM Tris-Acetat, 2 mM EDTA (pH 7,8)) nachgewiesen und daraus isoliert werden.
2. Klonierung von HPV 41 in Plasmid pUC 19
   Als Klonierungsvektor wurde das bekannte Plasmid pUC 19 (Yanish-Perron et al. (1985), Gene 33, 113-119) gewählt. Zirkular-geschlossene Doppelstrang DNA wurde nach Spaltung mit BamHI in pUC 19 einkloniert (Maniatis et al. (1982), Molecular Cloning: a Laboratory Manual; Cold Spring Harbor Laboratory Press, New York). Rekombinante Klone wurden im β-Galactosidase-Test (Messing et al. (1977), Proc. Nat. Acad. Sci. USA 74, 3642-3646) identifiziert und einligierte DNA-Stücke nach DNA Schnellextraktion analysiert (Birnboim, H.L. und Doly. (1979), Nucl. Acids Res. 7, 1513-1523).
   Zwei Rekombinanten wurden erhalten, von denen einer, K 10, eine Insertierung von 6,6 kb enthielt, der andere, K 6, eine solche von 0,98 kb. Die Homologie dieser klonierten Sequenzen ist durch Hybridisierung mit episomaler DNS aus Biopsiematerial nachgewiesen.
3. Physikalische Genomkarten von HPV 41
   Vom Vektor durch BamHI-Spaltung abgetrennte HPV 41 DNA wurde mit Restriktionsendonukleasen AccI, BglII, XbaI, SmaI, EcoRI, PstI und HincII verdaut und nach allgemein bekannten Methoden die entsprechenden physikalischen Genomkarten erstellt. Das Ergebnis ist in Fig. 1 zusammengefaßt, wobei die alleinige KpnI-Schnittstelle dazu diente, das HPV 41 Molekül zu linearisieren.
4. Vergleich mit anderen HPV
   Die DNA des HPV 41-Genoms wurde mit den DNA's von 40 erhältlichen HPV-Typen mittels DNA/DNA Hybridisierung unter verschiedener Stringenz verglichen (E.M. Southern (1975), J. Mol. Biol. 98, 503-517). Unter Bedingungen hoher Stringenz (Schmelztemperatur Tm -20°C) hybridisiert HPV 41 K 10-DNA teilweise mit HPV 29-DNA. Die DNA des K 6-Klones hybridisiert nur bei niedriger Stringenz (Tm -40°C) mit HPV 3- und 13-DNA.

Die Kolinearität mit HPV 8 aufgrund von Hybridisierungsversuchen zeigt Fig. 2.

In Kenntnis der HPV 8 DNA-Sequenz (Fuchs et al. (1986), J. Virol. 58, 626-634) sind die offenen Leseraster von HPV 41 ableitbar und damit die HPV 41 Proteine erhältlich über allgemein bekannte Methoden zur Subklonierung der Klone K 10 und K 6 mit anschließender Expression in prokaryontischen oder eukaryontischen Expressionssystemen.

Die Plasmide HPV 41 Klon K6 und K10 sind (in E. coli) am 3.7.1987 bei der Deutschen Sammlung für Mikroorganismen unter den Nummern DSM 4174P (für K6) und DSM 4175P (für K10) hinterlegt worden.

## Patentansprüche (Patentansprüche für folgende(n) Vertragsstaat(en): AT, BE, CH, DE, FR, GB, GR, IT, LI, LU, NL, SE)

1. HPV 41 DNA, gekennzeichnet durch die hinterlegten Klone DSM 4175 P und DSM 4174 P.

2. Diagnostikum, enthaltend DNA gemäß Anspruch 1 oder Teile dieser DNA.

3. Verwendung der DNA nach Anspruch 1 zur gentechnischen Expression von Proteinen.

4. Verfahren zur Diagnose von HPV 41-Infektionen, dadurch gekennzeichnet, daß man Biopsie- oder Abstrichmaterial mit einem Diagostikum nach Anspruch 2 in Kontakt bringt.

5. Verfahren zur Diagnose von HPV 41-Infektionen, dadurch gekennzeichnet, daß man die zu untersuchende RNA oder DNA mit DNA nach Anspruch 1 hybridisiert.

## Patentansprüche (Patentansprüche für folgende(n) Vertragsstaat(en): ES)

1. Verfahren zur Expression von HPV 41 DNA, dadurch gekennzeichnet, daß sie wie in den hinterlegten Klonen DSM 4175 P und DSM 4174 P vorhanden in geeignete Expressionsvektoren gebracht und in pro- oder eukaryontischen Zellen exprimiert wird.

2. Verfahren zur Herstellung von HPV 41 DNA, dadurch gekennzeichnet, daß sie wie in den Klonen DSM 4175 P und DSM 4174 P hinterlegt, in geeigneter Weise vermehrt und isoliert wird.

3. Verfahren zur Diagnose von HPV 41-Infektionen, dadurch gekennzeichnet, daß DNA hergestellt gemäß Anspruch 2 oder Teile davon eingesetzt werden.

4. Verfahren zur Diagnose von HPV 41-Infektionen, dadurch gekennzeichnet, daß man Biopsie- oder Abstrichmaterial mit DNA hergestellt nach Anspruch 2 oder Teilen dieser DNA in Kontakt bringt und mittels bekannter Hybridisierungstechniken die in dem Biopsie- oder Abstrichmaterial vorhandene DNA oder RNA bestimmt.

## Claims (Claims for the following Contracting State(s): AT, BE, CH, DE, FR, GB, GR, IT, LI, LU, NL, SE)

1. HPV 41 DNA, distinguished by the deposited clones DSM 4175 P and DSM 4174 P.

2. A diagnostic agent, containing DNA as claimed in claim 1, or parts of this DNA.

3. Use of the DNA as claimed in claim 1, for the recombinant expression of proteins.

4. A process for diagnosing HPV 41 infections, wherein biopsy or smear material is brought into contact with a diagnostic agent as claimed in claim 2.

5. A process for diagnosing HPV 41 infections, wherein the RNA or DNA being investigated is hybridized with DNA as claimed in claim 1.

## Claims (Claims for the following Contracting State(s): ES)

1. A process for expressing HPV 41 DNA, wherein the DNA, as available in the deposited clones DSM 4175 P and DSM 4174 P, is introduced into suitable expression vectors and expressed in prokaryotic or eukaryotic cells.

2. A process for preparing HPV 41 DNA, wherein the DNA, as deposited in the clones DSM 4175 P and DSM 4174 P, is replicated and isolated in a suitable manner.

3. A process for diagnosing HPV 41 infections, wherein DNA prepared as claimed in claim 2, or parts thereof, is/are employed.

4. A process for diagnosing HPV 41 infections, wherein biopsy or smear material is brought into contact with DNA prepared as claimed in claim 2, or parts of this DNA, and the DNA or RNA present in the biopsy or smear material is determined with the aid of known hybridization techniques.

## Revendications (Revendications pour l'(les) Etat(s) contractant(s) suivant(s): AT, BE, CH, DE, FR, GB, GR, IT, LI, LU, NL, SE)

1. ADN de HPV 41, caractérisé par les clones déposés DSM 4175 P et DSM 4174 P.

2. Agent de diagnostic, comprenant l'ADN selon la revendication 1 ou des fragments de cet ADN.

3. Utilisation de l'ADN selon la revendication 1 pour l'expression de protéines par génie génétique.

4. Procédé pour le diagnostic d'infections par HPV 41, caractérisé en ce que l'on met en contact un matériau de biopsie ou de frottis avec un agent de diagnostic selon la revendication 2.

5. Procédé pour le diagnostic d'infections par HPV 41, caractérisé en ce que l'on hybride l'ARN ou l'ADN devant être détecté avec l'ADN selon la revendication 1.

## Revendications (Revendications pour l'(les) Etat(s) contractant(s) suivant(s): ES)

1. Procédé pour l'expression d'ADN de HPV 41, caractérisé en ce qu'on l'insère dans des vecteurs d'expression appropriés et on l'exprime dans des cellules pro- ou eucaryotes, comme dans les clones disponibles déposés DSM 4175 P et DSM 4174 P.

2. Procédé pour la fabrication d'ADN de HPV 41, caractérisé en ce qu'on le multiplie et on l'isole de manière appropriée, comme dans les clones déposés DSM 4175 P et DSM 4174 P.

3. Procédé pour le diagnostic d'infections par HPV 41, caractérisé en ce que l'on utilise de l'ADN obtenu selon la revendication 2 ou des fragments de ce dernier.

4. Procédé pour le diagnostic d'infections par HPV 41, caractérisé en ce que l'on met en contact un matériau de biopsie ou de frottis avec l'ADN obtenu selon la revendication 2 ou des fragments de cet ADN et on détecte l'ADN ou l'ARN présent dans le matériau de biopsie ou de frottis au moyen de techniques connues d'hybridation.
